# EUROPÄISCHE PATENTSCHRIFT

(11) **EP 0 312 839 B1**
(45) Veröffentlichungstag und Bekanntmachung des Hinweises auf die Patenterteilung: **26.05.1993**
(21) Anmeldenummer: 88116529.4
(22) Anmeldetag: 06.10.1988
(51) Int. Cl.: C12N 7/06, A61K 39/275, A61K 39/205, A61K 39/155, A61K 39/15

(54) **Verfahren zur Herstellung von Paramunitätsinducern**
Method for the preparation of an inducer of non-specific immunity
Procédé pour la préparation d'une substance provoquant une immunité non spécifique

(30) Priorität: 17.10.1987 DE 3735277; 11.05.1988 DE 3816139
(43) Veröffentlichungstag der Anmeldung: 26.04.1989
(73) Patentinhaber: BAYER AG, 51368 Leverkusen (DE)
(72) Erfinder: Mayr, Anton, Prof. Dr. Dr., D-8000 München 50 (DE); Thein, Peter, Prof. Dr. Dr., D-8064 Oberzeitlbach (DE); Strube, Walter, Dr., D-5000 Köln 40 (DE)

(56) Entgegenhaltungen:
- EP-A- 0 037 441
- EP-A- 0 124 506
- WO-A-86/00811
- DE-A- 1 021 133
- BIOLOGICAL ABSTRACTS, Band 71, 1981, Zusammenfassung Nr. 52535, Philadelphia, PA, US; P. THEIN et al.: "Experiences with using a paramuniring inducer PIND-AVI in equine practice",& ZENTRALBL VETERINAERMED REIHE B 27(6): 49 9-512, 1980
- BIOLOGICAL ABSTRACTS, Band 73, 1982, Zusammenfassung Nr. 75911, Philadelphia, PA, US; P. THEIN et al.: "Comparative studies on the activity of paraimmunity inducer phytohemagglutinin PMITOGEN and rhinopneumonites virus on the peripheral leukocytes of the horse",& ZENTRALBL VETERINAERMED REIHE B 28(6): 432-449, 1981
- JOURNAL OF GENERAL MICROBIOLOGY, Band 38, 1965, Seiten 39-45; H.T. ZWARTOUW et al.: "Antigens from vaccinia virus particles"
- Mayr et al (1989) J. Vet. Med. B. 36, 81-99

## Beschreibung

Die vorliegende Erfindung betrifft ein Verfahren zur Herstellung von Paramunitätsinducern aus Viren durch eine zweistufige Behandlung, die zur Inaktivierung unter gleichzeitiger Erniedrigung der Antigenität führt, die paramunisierenden Aktivitäten aber nicht zerstört.

Unter Paramunisierung wird nach A. Mayr der Erwerb eines schnell entstehenden, unterschiedlich lange anhaltenden, nicht erreger- und nicht antigenspezifischen (paraspezifischen) Schutzes eines Individuums gegenüber einer Mehrzahl ganz unterschiedlicher Infektionen, Toxine und Antigene bezeichnet. Der paraspezifische Schutz (Paramunität) entsteht durch die Stimulierung des paraspezifischen Teiles des zellulären und humoralen Immunsystems.

Paramunität kann auf natürliche Weise im Verlauf eines Infektionsgeschehens oder medikamentös durch Paramunitätsinducer, Immunmodulatoren, biological response modifiers (BRM) etc. erworben werden. Bekannte Paramunitätsinducer sind z. B. inaktivierte Geflügelpocken-, Parapocken- oder Orthopockenviren [Tierärztl. Praxis 14, S. 237 - 244 (1986)].

Die Inaktivierung bekannter Paramunitätsinducer erfolgt durch Gammastrahlen, durch Erwärmen oder durch chemische Verfahren (DE-OS 2 714 665). Ein zweistufiges Inaktivierungsverfahren ist bis jetzt noch nicht zur Herstellung von Paramunitätsinducern bekannt.

Die verwendeten Methoden sind als Technik per se nicht neu. Sie wurden bisher in der Virologie jedoch ausschließlich zu anderen Zwecken eingesetzt. Zwartouw et. al. [J. gen. Microbiol. 38, 39 - 45 (1965)] isolierten mit Hilfe von Wärme und Alkalisierung präzipitierende Antigene aus Vacciniapartikeln. Diese Arbeiten galten nicht einer Inaktivierung, sie hatten das Ziel, Antigene nach unterschiedlicher Vorbehandlung des Ausgangsmaterials mit Serum, in dem Antikörper gegen das komplette Virus enthalten war, um Überstand der behandelten Virussuspensionen nachzuweisen.

In dem gewählten System konnten diese Autoren lediglich nachweisen, daß es nach der chemisch thermischen Behandlung des Vaccinia-Virus zu quantitativ schwächer ausgeprägter Antigen-Antikörper-Reaktion kam.

Die bisherigen Erfahrungen mit Paramunitätsinducern aus Pockenviren und Paramyxoviren beweisen, daß für die Wirksamkeit derartiger Inducer 2 Kriterien wichtig sind:
1. eine schonende Inaktivierung und
2. eine Reduktion der Gesamtantigenität bei gleichzeitigem Erhalt der paramunisierenden Aktivitäten.

Es wurde gefunden, daß man Paramunitätsinducer erhält, indem man den pH-Wert von Virussuspensionen von Viren, die Paramunität hervorrufen, auf Werte zwischen 8 und 11 einstellt und anschließend auf 50 - 60°C über 40 - 120 Minuten erwärmt.

Überraschend ist, daß durch eine Kombination von Alkalisierung und Erwärmung die Forderung nach Inaktivierung der Viren und Senkung der Gesamtantigenität unter Erhalt der paramunisierenden Aktivitäten nicht nur in optimaler Weise erfüllt wird, sondern sich hierdurch zusätzliche Vorteile für die Herstellung von Paramunitätsinducern ergeben. Diese sind:
1. Einfache Durchführung des Inaktivierungsverfahrens ohne Belastung durch Strahlen oder toxische Chemikalien.
2. Durch die verringerte Antigenität wird die Gefahr der Sensibilisierung nach wiederholter Anwendung verringert.
3. Verbesserte Wirtschaftlichkeit durch die Vereinfachung des Herstellungsverfahrens.

Das Verfahren wird durchgeführt, indem man den pH-Wert der Virussuspension auf Werte zwischen etwa 8 - 11, bevorzugt zwischen etwa 9 - 10, einstellt.

Zur Einstellung dienen wäßrige anorganische Alkalihydroxide wie z. B. Natrium-, Kaliumhydroxid, Carbonate wie z. B. Natrium- oder Kaliumcarbonat, Hydrogencarbonate wie z. B. Natrium- oder Kaliumhydrogencarbonat.

Anschließend wird thermisch inaktiviert durch Erhitzen auf etwa 50 - 60°C über 40 - 120 Minuten, bevorzugt ist Erhitzen auf 55 - 60°C, insbesondere auf etwa 56°C über 60 - 120 Minuten.

Besonders bevorzugt wird zur Inaktivierung bei etwa pH 10 und bei etwa 56°C über etwa 60 - 120 Minuten gearbeitet.

Als Virusarten zur Herstellung von Paramunitätsinducern, für die die erfindungsgemäße Inaktivierung besonders geeignet ist, seien genannt:
Pockenviren, insbesondere vom Genus Avipox, besonders bevorzugt Spezies gallinae, attenuierter Impfstamm HP-1, 438. Passage in Hühnerembryofibroblastenkulturen (FHE). (Der Stamm ist bekannt z. B. aus Collegium Veterinarium 1978, S. 35 ff. Der Virusstamm ist unter der Hinterlegungsbezeichnung "Mayr-Stickl-Avipox-Interferon-Inducer" bei der Landesimpfanstalt Nordrhein-Westfalen, Abteilung Viruszüchtung und -prüfung, hinterlegt. Er ist für die Abgabe an die Öffentlichkeit freigegeben.);
vom Genus Parapoxvirus besonders bevorzugt die Spezies Orfvirus, der attenuierte Stamm C 1701, 138. Passage in Lammnieren- bzw. embryonalen bovinen Rinderlungenkulturen (Der Stamm ORF D 1701 ist bekannt z. B. aus Tierärztl. Praxis 14, 237 - 244 (1986)] ; Laborbezeichnung für den daraus hergestellten Paramunitätsinducer: PIND-ORF. Der Stamm wurde am 1.9.81 bei der Bayerischen Landesimpfanstalt, Am Neudeck 1, 8000 München 95, als Impfstamm hinterlegt. Der Stamm wurde am 28.4.1988 beim Institut Pasteur, Collection Nationale de Cultures de Microorganismes, 25 rue du Docteur Roux, 75724 Paris, unter der Bezeichnung CNCM I-751 hinterlegt;
vom Genus Orthopoxvirus besonders bevorzugt die Spezies Vacciniavirus, der attenuierte Stamm MVA, 530. Passage des Vacciniavirus CVA in FHE-Kulturen [Der Stamm MVA ist von A. Mayr beschrieben im Zbl. Bakt. Hyg. I. Abt. Orig. B, 167, 367 (1978)]. Er wurde zur Vorimpfung gegen Menschenpocken vom Paul-Ehrlich-Institut, Frankfurt, zugelassen und ist hinterlegt im Institut für Mikrobiologie, Düsseldorf.

Zur Herstellung von Paramunitätsinducern mit der erfindungsgemäßen Inaktivierung eignen sich aber auch andere Virusarten, wie z. B. Paramyxoviren, Reoviren, Herpesviren.

Die Vermehrung der Viren erfolgt zunächst in an sich bekannter Weise auf geeigneten Zellkulturen, z. B. auf Zellkulturen von Säugetier- oder Vogelgewebezellen. Es kann dabei in Einschichtzellkulturen oder Suspensionskulturen oder im bebrüteten Hühnerei gearbeitet werden.

Als Nährmedien für die Einschicht- bzw. Suspensionskulturen finden die bekannten Medien z. B. nach Earle, Eagle, Hanks Verwendung.

Das Virus wird nach bekannten Verfahren geerntet und gegebenenfalls gereinigt. Die Virusernte wird dann dem erfindungsgemäßen Inaktivierungsverfahren unterworfen.

Die inaktivierte Virusernte wird anschließend gekühlt oder gefriergetrocknet und gelagert bzw. zur Herstellung eines Paramunitätsinducers verwendet. Dazu werden Präparate hergestellt, die über die Schleimhaut z.B. oral oder intranasal und parenteral, z.B. intramuskulär, subkutan, appliziert werden können.

Die gemäß den folgenden Beispielen hergestellten Paramunitätsinducer werden lyophilisiert und dienen in dieser Form als Ausgangsprodukt zur Herstellung entsprechender Arzneimittel. Vor ihrer Verwendung wird das Lyophilisat entsprechend dem Ausgangsvolumen mit üblichen pharmazeutischen Trägern, vorzugsweise mit sterilem, pyrogenfreien Aqua dest., versetzt. In dieser Zubereitungsform eignet es sich sowohl für die parenterale (z. B. intramuskulär, subcutan) als auch für die lokale Verabreichung.

Die Paramunitätsinducer können auch in andere pharmazeutische Träger eingearbeitet werden. Hierbei muß berücksichtigt werden, daß die Sterilität und Haltbarkeit, z. B. durch proteolytische Bestandteile des Trägers, nicht beeinträchtigt werden.

In der Prophylaxe werden Paramunitätsinducer 1 bis 3 mal im Abstand von 24 Stunden verabreicht. Beim therapeutischen Einsatz werden je nach Schwere der Erkrankung 1 bis 3 Behandlungen pro Tag über 3 bis 5 Tage durchgeführt. Die Dosis richtet sich nach der Größe der betreffenden Spezies und nach der Applikationsform (z. B. Rind parenteral: 4 ml; Welpe (Hunde) parenteral: 0,5 ml).

### Beispiel 1

Als Ausgangsmaterial dient der attenuierte, plaquegereinigte (3 Passagen) Geflügelpocken-Virusstamm HP-1 in der 441. Kulturpassage in Hühnerembryofibroblastenkulturen (FHE) mit einem Infektiositätstiter von 10^{7,5} KID₅₀/ml. Für die Anzüchtung der FHE-Kulturen dient Earle MEM mit einem Zusatz von 3 % fötalem Kälberserum, als Erhaltungsmedium (Virusmedium) Earle MEM ohne Zusätze. Mit dem Virusmaterial werden ca. 90 % dichte FHE-Monolayer-Kulturen beimpft (z. B. pro Rouxschale je 200 ml Medium 0,5 ml Virussuspension). Ca. 24 Stunden p. inf. bzw. wenn die Zellkultur zu 80 - 100 % virusspezifisch verändert ist (Granulation, Abkugelung, Lysis der Zellen), wird der noch vorhandene Zellrasen durch Schütteln der Kulturen abgelöst und das Virus sowie das zellhaltige Medium mit Ultraschall behandelt (Branson Sonifier 15 min.), um die noch intakten Zellen aufzuschließen. Der Zelldetritus wird anschließend niedertourig abzentrifugiert.

Der Virusernte werden zur Kontrolle der Sterilität und Infektiosität Proben entnommen. Nur Virusernten, die frei von Kontaminationen (Viren, Bakterien, Pilze, Mykoplasmen) sind und einen Infektiositätstiter von mindestens 10^{7,25}KID₅₀/ml besitzen, werden weiter bearbeitet. Ernten, die diesen Ansprüchen nicht genügen, werden verworfen.

Die Virusernte wird bis zur weiteren Bearbeitung bei +4° oder -60°C gelagert.

Vor Beginn der zweistufigen Inaktivierung wird das Virusmaterial auf 37°C angewärmt. In der ersten Inaktivierungsstufe wird die Virussuspension mit 1n NaOH auf einen pH-Wert von 10.0 eingestellt. Die alkalisierte Suspension wird danach sofort in ein Wasserbad mit 56°C verbracht. Um eine gleichmäßige Durchmischung und Erwärmung zu erzielen, wird die Virussuspension mit Hilfe eines Magnetrührers und das Wasserbad mit einem üblichen Rührwerk ständig durchmischt. Die thermische Inaktivierung wird über 60 Minuten durchgeführt.

Nach dieser Zeit wird durch Folgepassagen belegt, daß keine Restinfektiosität mehr nachweisbar ist.

Der so gewonnene Paramunitätsinducer wird als PIND-AVT^{t} (therm.) bezeichnet und bei Temperaturen von +4°C oder -60° C gelagert. Er kann auch in gefriergetrockneter Form aufbewahrt werden.

Die Reinheit, Spezifität und Unschädlichkeit des erfindungsgemäß erhaltenen Paramunitätsinducers PIND-AVI^{t} wird durch entsprechende Kontrolluntersuchungen überprüft.

Die Prüfung auf Spezifität, Reinheit und Unschädlichkeit erfüllt die Forderungen des Europ. Arzneibuches in folgenden Kriterien:
1. Elektronenmikroskopische Kontrolle mit den üblichen Methoden zeigen im Präparat massenhaft typische, z. T. leere Pockenpartikel. Es ist frei von anderen mikrobiellen Strukturen.
2. Die üblichen Kontrollen auf mikrobielle Kontamination zeigen, daß das Produkt frei von anderen Viren, Bakterien, Pilzen und Mykoplasmen ist.
3. Nach den Richtlinien des Europäischen Arzneibuchs wurde Toxizität, Teratogenität, Pyrogenität geprüft und keine positiven Ergebnisse festgestellt.
4. Kontrolle auf den Gehalt an nicht inaktiviertem Restvirus: hierfür wird mit dem fertigen Paramunitätsinducer in üblicher Weise eine Titrierung in FHE-Röhrchenkulturen durchgeführt und der Infektiositätstiter mit Hilfe des auftretenden cytho - patischen Effekts bestimmt. In weiteren 3 Folgepassagen liegt der Restvirusgehalt bei 0.

Zum Nachweis der Wirksamkeit wird der Paramunitätsinducer nach der folgenden Methode untersucht:
Als in vivo-Nachweis wird das VSV-Infektionsmodell in der Babymaus verwendet [J. Vet. Med. B, 33, 321 - 339 (1986)]. Es kann gleichzeitig zur Bestimmung der Wirkungseinheiten (WE/ml) dienen.

In der Tabelle 1 ist die Titrierung der Wirksamkeit eines PIND-AVI^{t} im VSV-Modell dargestellt. Eine derartige Titrierung dient der Bestimmung der Wirkungseinheiten (WE/ml) einer Charge: diejenige Verdünnnungsstufe, bei der noch ein Wirkungsindex von mindestens 20 erzielt wird, enthält 1 WE pro 0,1 ml (Injektionsdosis/pro Maus) bzw. 10 WE/ml (im Beispiel: 1 : 16 Endtiter; die Charge enthält 160 WE/ml.).

**Tabelle 1**

| Titrierung der paramunogenen Wirksamkeit von PIND-AVI^{t} im VSV-Infektionsmodell | | |
|---|---|---|
| Präparat | Verdünnungsstufe | Wirkungsindex |
| PIND-AVI^{t} | 1 : 4 | 48 |
| | 1 : 8 | 45 |
| | 1 : 16 | 34 |
| | 1 : 32 | 13 |

Als in-vitro-Nachweis der Wirksamkeit des erfindungsgemäßen Paramunitätsinducers wird
1. die Aktivität peritonealer NK-Zellen 24 Stunden nach Behandlung mit PIND-AVI^{t} im 4 Stunden-Chromium 51-Freisetzungstest verwendet [J. Vet. Med. B, 33, 321 - 339 (1986)]. Mäusen werden hierfür 24 Stunden vor der Zellgewinnung 0,2 ml PIND-AVI^{t} appliziert. Die Ergebnisse aus diesem Test zeigen eine dosisabhängige Wirkung des PIND-AVI^{t}.

In einem in-vitro-Test wird der Einfluß von PIND-AVI^{t} auf die koloniestimulierende Aktivität im Serum von Mäusen 8 Stunden nach der Behandlung getestet. Die Ergebnisse aus diesem CSA-Test zeigen eine dosisabhängige Wirkung des PIND-AVI^{t}.

### Beispiel 2

Als Ausgangsmaterial für die Herstellung eines Paramunitätsinducers wird das attenuierte Parapoxvirus ovis (ORF, Ecthyma des Schafes), Stamm D 1701, in der 138. Kulturpassage (Infektiositätstiter: 10^{7,75}KID₅₀/ml) in Lammnieren- bzw. embryonalen Rinderlungenkulturen verwendet. Herstellung und Inaktivierung der Virusernten erfolgen gemäß Beispiel 1, jedoch mit einer verlängerten thermischen Inaktivierung von 2 Stunden. Nach ca. 2 Stunden ist vollständige Inaktivierung oder Virusinfektiosität erreicht.

Die Prüfungen auf Reinheit, Unschädlichkeit, Spezifität und Wirksamkeit erfolgen wie in Beispiel 1 beschrieben. Es werden die gleichen Ergebnisse wie im Beispiel 1 erzielt.

### Beispiel 3

Die Herstellung des Paramunitätsinducers sowie dessen Prüfung auf Reinheit und Wirksamkeit erfolgt gemäß Beispiel 1, jedoch mit dem attenuierten Orthopoxvirus commune, Vaccinia virus, Stamm MVA. Das nach diesem Verfahren inaktivierte Virus hat paramunisierende Eigenschaften.

### Beispiel 4

Die Herstellung des Paramunitätsinducers sowie dessen Prüfung auf Reinheit und Wirksamkeit erfolgt gemäß Beispiel 1, jedoch mit dem lentogenen Newcastle Desease Virus (Paramyxovirus), Stamm Hitchner B₁. Das nach diesem Verfahren inaktivierte Virus hat paramunisierende Eigenschaften.

### Beispiel 5

Die Herstellung des Paramunitätsinducers sowie dessen Prüfung auf Reinheit und Wirksamkeit erfolgt gemäß Beispiel 1, jedoch mit dem Reovirus Serotyp 3. Das nach diesem Verfahren inaktivierte Virus hat paramunisierende Eigenschaften.

### Beispiel 6

Um die Verwendungsmöglichkeiten und die Wirksamkeit des gemäß Beispiel 1 hergestellten Paramunitätsinducers zu demonstrieren, sind Beispiele für die therapeutische und prophylaktische Anwendung in den folgenden Tabellen 2 und 3 zusammengestellt.

Die Verwendung von PIND-AVI^{t} in einem Doppelblindversuch bei trächtigen Hündinnen und deren Würfen verdeutlicht, daß durch die prophylaktische Anwendung dieses Präparates das infektiöse Welpensterben, das in der Regel durch eine hohe Mortalität in der ersten Lebenswoche gekennzeichnet ist, wirksam unter Kontrolle gebracht werden kann (Tabelle 2).

Der Doppelblindversuch in einem Kälbermastbetrieb, in dem die Behandlung mit PIND-AVI^{t} sofort nach dem Eintreffen der Kälber in der Mästerei durchgeführt worden ist (2 x je 4 ml i. m. im Abstand von 24 Stunden), zeigt deutlich, daß das Präparat eine ausgezeichnete prophylaktische Wirksamkeit gegenüber Infektionen unterschiedlicher Genese besitzt.

Wie aus den Daten hervorgeht, können durch den Einsatz des Paramunitätsinducers Infektionskrankheiten unterschiedlicher Genese in ihrem Verlauf gestoppt, gemildert oder überhaupt am Ausbruch gehindert werden.

Sie beweisen ferner die Unschädlichkeit der erfindungsgemäßen Paramunitätsinducer. Es wurden keine Nebenreaktionen beobachtet.

**Tabelle 2**

| Wirksamkeit von PIND-AVI^{t} bei der Prophylaxe des infektiösen Welpensterbens | | |
|---|---|---|
| Krankheitsbild und Zahl der behandelten Fälle | Behandlungsmodus | Ergebnisse |
| 42 neugeborene Welpen aus 10 Würfen; behandelt | Mütterhündin in der letzten Woche der Trächtigkeit: 2 x 1 ml i. m. | alle 42 Welpen gesund aufgezogen |
| | neugeborene Welpen: je 0,5 ml i. m. | |
| | 1. sofort nach der Geburt | |
| | 2. 12 - 24 Stunden p. part. | |
| 18 unbehandelte Wurfgeschwister | | 6 aufgezogen, |
| | | 12 innerhalb der ersten Lebenswoche gestorben |

**Tabelle 3**

| Wirksamkeit von PIND-AVI^{t} bei der Prophylaxe der Crowding Disease in der Kälbermast | | |
|---|---|---|
| Versuchsgruppe | erkrankte Kälber | gestorbene Kälber |
| 32 Kälber, PIND-AVI^{t}-behandelt | 2 | 0 |
| 32 Kälber, unbehandelt | 15 | 4 |

## Patentansprüche

1. Verfahren zur Inaktivierung von Virussuspensionen, die zur Herstellung von Parmunitätsinducers dienen, das dadurch gekennzeichnet ist, daß man die Virusernte einer zweistufigen Inaktivierung durch Erhöhung des pH-Wertes und durch Erwärmen unterwirft.

2. Verfahren gemäß Anspruch 1, dadurch gekennzeichnet, daß man als Virusernte ein Parapockenvirus der Spezies Orfvirus oder Stomatitis papulose-Virus einsetzt.

3. Verfahren gemäß Anspruch 1, dadurch gekennzeichnet, daß die Virusernte zunächst auf einen pH-Wert zwischen 8 und 11 eingestellt wird und anschließend für 40 bis 120 Minuten auf Temperaturen zwischen 50 und 60°C erhitzt wird.

4. Verfahren gemäß Anspruch 1, dadurch gekennzeichnet, daß die Virusernte zunächst auf einen pH-Wert zwischen 9 und 10 eingestellt wird und anschließend für 60 bis 90 Minuten auf Temperaturen zwischen 55 und 60°C erhitzt wird.

5. Verfahren gemäß Anspruch 1, dadurch gekennzeichnet, daß es mit Geflügelpockenviren durchgeführt wird.

6. Verfahren gemäß Anspruch 1, dadurch gekennzeichnet, daß es mit Paramyxoviren oder Reoviron durchgeführt wird.

## Claims

1. Process for the inactivation of virus suspensions used for the preparation of inducers of non-specific immunity, which process is characterised in that the virus harvest is subjected to a two-stage inactivation by increasing the pH and by heating.

2. Process according to Claim 1, characterised in that the virus harvest used is a parapoxvirus of the species orfvirus or stomatitis papulose virus.

3. Process according to Claim 1, characterised in that the virus harvest is first adjusted to a pH between 8 and 11 and then heated at temperatures between 50 and 60°C for 40 to 120 minutes.

4. Process according to Claim 1, characterised in that the virus harvest is first adjusted to a pH between 9 and 10 and then heated at temperatures between 55 and 60°C for 60 to 90 minutes.

5. Process according to Claim 1, characterised in that it is carried out with fowl pox viruses.

6. Process according to Claim 1, characterised in that it is carried out with paramyxoviruses or reoviruses.

## Revendications

1. Procédé d'inactivation de suspensions de virus, qui sert à la préparation de substances inductrices d'immunité non spécifique, caractérisé en ce qu'on soumet la récolte de virus à une inactivation en deux étapes par élévation de la valeur du pH et par chauffage.

2. Procédé selon la revendication 1, caractérisé en ce qu'on intilise comme récolte de virus un parapoxvirus de l'espèce du virus de l'orf ou din virus de la stomatite papuleuse.

3. Procédé selon la revendication 1, caractérisé en ce qu'on amène d'abord la récolte de virus à un pH compris entre 8 et 11, et qu'on la chauffe ensuite pendant 40 à 120 minutes à des températures comprises entre 50 et 60°C.

4. Procédé selon la revendication 1, caractérisé en ce qu'on amène d'abord la récolte de virus à un pH compris entre 9 et 10, et qu'on la chauffe ensuite pendant 60 à 90 minutes à des températures comprises entre 55 et 60°C.

5. Procédé selon la revendication 1, caractérisé en ce qu'il est effectué avec des poxvirus aviaires.

6. Procédé selon la revendication 1, caractérisé en ce qu'il est effectué avec des paramyxovirus ou des réovirus.
